(19) Europäisches Patentamt
European Patent Office
Office européen des brevets

(11) **EP 1 663 162 B1**

(12) # EUROPÄISCHE PATENTSCHRIFT

(45) Veröffentlichungstag und Bekanntmachung des Hinweises auf die Patenterteilung:
**28.05.2008 Patentblatt 2008/22**

(51) Int Cl.:
***A61K 9/16*** (2006.01)          ***A61K 9/50*** (2006.01)

(21) Anmeldenummer: **04764642.7**

(22) Anmeldetag: **31.08.2004**

(86) Internationale Anmeldenummer:
**PCT/EP2004/009676**

(87) Internationale Veröffentlichungsnummer:
**WO 2005/023223 (17.03.2005 Gazette 2005/11)**

(54) **ORAL ZU APPLIZIERENDE DARREICHUNGSFORM FÜR SCHWERLÖSLICHE SAURE UND AMPHOTERE WIRKSTOFFE**

ORALLY ADMINISTRABLE DOSAGE FORM FOR POORLY SOLUBLE ACIDS AND AMPHOTERIC ACTIVE INGREDIENTS

FORME GALENIQUE A ADMINISTRATION ORALE POUR SUBSTANCES ACTIVES ACIDES ET AMPHOTERES DIFFICILEMENT SOLUBLES

(84) Benannte Vertragsstaaten:
**AT BE BG CH CY CZ DE DK EE ES FI FR GB GR HU IE IT LI LU MC NL PL PT RO SE SI SK TR**

(30) Priorität: **05.09.2003 DE 10341414**

(43) Veröffentlichungstag der Anmeldung:
**07.06.2006 Patentblatt 2006/23**

(73) Patentinhaber:
• **Boehringer Ingelheim International GmbH 55216 Ingelheim am Rhein (DE)**
Benannte Vertragsstaaten:
**AT BE BG CH CY CZ DK EE ES FI FR GB GR HU IE IT LI LU MC NL PL PT RO SE SI SK TR**
• **Boehringer Ingelheim Pharma GmbH & Co.KG 55216 Ingelheim am Rhein (DE)**
Benannte Vertragsstaaten:
**DE**

(72) Erfinder:
• **BRICKL, Rolf-Stefan 88447 Warthausen (DE)**
• **BRAUNS, Ulrich 88400 Biberach (DE)**
• **FRIEDL, Thomas 88416 Ochsenhausen (DE)**

(74) Vertreter: **Hammann, Heinz et al Boehringer Ingelheim Pharma GmbH & Co. KG Binger Strasse 173 55216 Ingelheim am Rhein (DE)**

(56) Entgegenhaltungen:
**US-A- 4 438 091          US-B1- 6 436 438**

**Beschreibung**

Die Erfindung betrifft eine oral zu applizierende Darreichungsform für saure und amphotere Wirkstoffe mit einem pH-abhängigen Löslichkeitsverhalten und deren Salze.

Als "Wirkstoff" im Sinne dieser Erfindung wird jede pharmakologisch wirksame Verbindung angesehen, die (als solche oder als eines ihrer pharmazeutisch annehmbaren Salze) eine schwache Säure ist oder sich amphoter verhält und im Bereich von pH 1 bis pH 11 ein pH-abhängiges Löslichkeitsverhalten (mit besserer Löslichkeit unter basischen Bedingungen und schlechterer Löslichkeit unter neutralen und/oder sauren Bedingungen) zeigt. Bevorzugt sind saure Wirkstoffe. Bei solchen Wirkstoffen kann nämlich die Bioverfügbarkeit bei oraler Gabe von dem pH-Wert im Magen-Darm-Trakt abhängig sein. Vorzugsweise weisen Wirkstoffe im Sinne dieser Erfindung in wässrigen Lösungen bei höheren pH-Werten eine verhältnismäßig hohe Sättigungslöslichkeit auf, während sie bei pH-Werten von ca. 5 gemäß der Definition des europäischen Arzneibuches praktisch unlöslich sind (Sättigungslöslichkeit kleiner als 100 $\mu$g/ml).

Die erfindungsgemäße orale Darreichungsform kann als Wirkstoff beispielsweise Telmisartan, Meloxicam, DT-TX 30 SE, BIBV 308 SE (Terbogrel), AGEE 623 (Repaglinide), Gliquidon oder Glibenclamide oder ein physiologisch unbedenkliches Salz davon enthalten. Zu den physiologisch unbedenklichen Salzen gehören beispielsweise das Natrium-, Kalium-, Calcium- und Ammoniumsalz sowie Salze mit Diethanolamin, Meglumin, Lysin, Arginin, Ethanolamin, Piperazin oder Triethanolamin.

Die Löslichkeit einer Verbindung kann bestimmt werden, indem man einen Überschuss der Verbindung bei Raumtemperatur in dem jeweiligen Medium dispergiert und für einen definierten Zeitraum (ca. 1 bis 24 h) bis zur Einstellung des Gleichgewichts kräftig schüttelt. Nach Filtration wird in dem klaren Filtrat der pH-Wert und mittels Spektralphotometrie oder einem anderen geeigneten analytischen Verfahren die Konzentration der gelösten Substanz bestimmt.

Das pH-abhängige Löslichkeitsverhalten des Wirkstoffes kann je nach Dosis dazu führen, dass dieser bei oraler Verabreichung konventionell zusammengesetzter fester Arzneiformen erst in tieferen Darmabschnitten des Patienten vollständig in Lösung gehen kann. Dies führt dann zu deutlich verzögerter, teilweise auch unvollständiger Resorption, d.h. die therapeutische Sicherheit ist nicht gewährleistet.

Der Einfluß der Dosis des jeweiligen Wirkstoffs auf seine Bioverfügbarkeit läßt sich quantitativ mittels des Konzepts der (dimensionslosen) Dosenumber (Do) beschreiben. Die Dosenumber wird definiert als:

$$Do = (Mo \; / \; Vo) \; / \; Cs \; ,$$

wobei

Mo = Dosis (mg),
Vo = vorhandenes Flüssigkeitsvolumen (ml) und
Cs = Sättigungslöslichkeit (mg / ml).

Gemäß einer heute üblichen Annahme beträgt das Flüssigkeitsvolumen im Magen nach Einnahme einer Darreichungsform ca. 250 ml. (Löbenberg, R., Amidon, G.L.: Modern bioavailability, bioequivalence and biopharmaceutics classification system. New scientific approaches to international regulatory standards (Eur. J. Pharm. Biopharm. 50 (2000) 3-12).

Bei Dosierungen, die eine Dosenumber von kleiner als 1 ergeben, treten keine Löslichkeitsprobleme auf. Erst wenn die kritische Dosenumber von 1 überschritten wird, kann es zu relevanten Verschlechterungen des Löslichkeit und damit zur Abnahme der Bioverfügbarkeit kommen. In der Regel beginnt die eigentliche Problemzone jedoch erst bei Dosierungen, die eine Dosenumber deutlich höher als 1 ergeben, da zumindest Teile der gelösten Substanz durch den Resorptionsvorgang ständig aus dem Gleichgewicht entfernt werden.

Die in der erfindungsgemäßen oralen Darreichungsform enthaltenen Wirkstoffe weisen für die Dosenumber bezogen auf die Löslichkeit im Bereich von pH 1 bis pH 7 für saure bzw. im pH-Bereich von 3 bis 7 für amphotere Wirkstoffe einen Wert von deutlich über 1 auf, d.h. für die erfindungsgemäße orale Darreichungsform ist sowohl das Ausmaß der pH-Abhängigkeit der Löslichkeit des Wirkstoffs als auch die Höhe der Wirkstoffdosis von Interesse. Die erfindungsgemäße Darreichungsform eignet sich besonders für Wirkstoffe, die unter pH 6 gemäß der oben definierten Dosenumber schlecht löslich sind.

Eine generelle Dosiserhöhung zur Kompensation der verminderten Bioverfügbarkeit in Patienten ist wegen der Wirkstoffverschwendung sowie wegen der erhöhten Belastung des Patienten und des damit verbundenen Risikos z.B. von Nebenwirkungen häufig unerwünscht bzw. aufgrund der Arzneimittelsicherheit überhaupt nicht möglich. Außerdem führt bei schwerlöslichen Wirkstoffen eine Erhöhung der Dosis nicht unbedingt zu den erwarteten Plasmaspiegeln: Bei DT-TX 30 wurden in einer Phase I Studie bei einer Dosisteigerung von 100 auf 600 mg nur ca 50 % höhere Plasmaspiegel erhalten, mit 200 mg wurden gleich hohe Spiegel wie bei 600 mg erreicht.

Aufgabe der Erfindung ist es, eine oral zu applizierende pharmazeutische Zusammensetzung für Wirkstoffe mit pH-abhängigeri Löslichkeitsverhalten bereitzustellen, die die weitgehend pH-unabhängige Bioverfügbarkeit des Wirkstoffs gewährleistet.

Überraschenderweise wurde nun gefunden, dass der Einsatz pharmazeutisch akzeptabler anorganischer oder organischer Basen mit einer Wasserlöslichkeit von größer als 1 g / 250 ml bei 20° C, vorzugsweise von größer als 1 g / 160 ml bei 25 °C, in festen oralen Darreichungsformen eine hinreichende Bioverfügbarkeit von Wirkstoffen mit pH-abhängigen Löslichkeitsverhalten gewährleisten kann. Bevorzugt sind die organischen Basen sowie Mischungen aus anorganischen und organischen Basen.

Pharmazeutisch geeignete Basen im Sinne dieser Erfindung sind beispielsweise Natriumhydroxid (NaOH), Kaliumhydroxid (KOH), Calciumhydroxid [$Ca(OH)_2$], Natriumcarbonat ($Na_2CO_3$), Ammoniak, Diethanolamin, Meglumin, Lysin, Arginin, - Ethanolamin, Piperazin, Trometamol und Triethanolamin. Besonders geeignet im Sinne dieser Erfindung sind NaOH, KOH, $Ca(OH)_2$, Ammoniak, Diethanolamin, Meglumin, Lysin und Arginin. Ganz besonders bevorzugt sind Meglumin, Lysin, Arginin und Trometamol. Vorteil der anorganischen Basen ist das geringe Molekulargewicht, das Formulierungen mit sehr hohen Wirkstoffgehalten ermöglicht, wobei das Verhältnis Base zu Wirkstoff so gewählt werden muß, dass keine Reizungen im Magen oder Darm infolge zu hoher pH-Werte auftreten. Gegebenenfalls kann man auch anorganische und organische Basen so kombinieren, dass physiologisch unbedenkliche pH-Werte von maximal 12 resultieren.

Zahlreiche Wirkstoffe zeigen in Gegenwart von Basen und Spuren von Wasser eine mehr oder weniger stark ausgeprägte Neigung zu hydrolytischer Zersetzung. In Einzelfällen kann es auch zu einer direkten chemischen Umsetzung zwischen dem Wirkstoff und Basen, z.B. zu einer Esterbildung, kommen. Zur Entwicklung eines lagerstabilen Produktes ist es daher vorteilhaft, in der Darreichungsform die Base räumlich vom Wirkstoff zu trennen. Erst nach Applikation der Darreichungsform geht dann die Base in Lösung und erzeugt ein basisches Mikroklima, in dem sich der Wirkstoff auflösen kann.

Eine weitere Aufgabe der Erfindung besteht daher darin, die unerwünschten Wechselwirkungen zwischen Base und Wirkstoff trotz Verwendung einer Base zur Löslichkeitsverbesserung zu vermeiden.

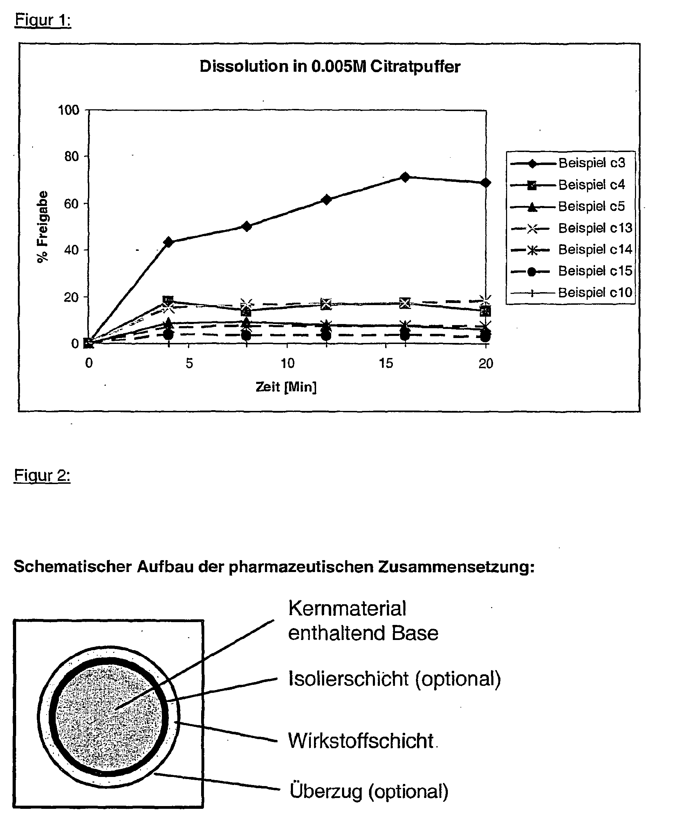

Für die bevorzugte räumliche Trennung von Wirkstoff und Base sind multipartikuläre Darreichungsformen, in denen die einzelnen Partikel wie in Figur 2 aufgebaut sind, besonders geeignet.

Figur 2 stellt den schematischen Aufbau der pharmazeutischen Zusammensetzung anhand eines Schnitts durch ein für die Herstellung der erfindungsgemäßen pharmazeutischen Zusammensetzung geeignetes Pellet dar. Der annähend kugelförmige/sphärische Kernbereich dieses Pellets enthält bzw. besteht aus einer oder mehrerer pharmazeutisch akzeptablen anorganischen oder organischen Basen. Dann folgt gegebenenfalls eine Schicht, die den Base-Kern von der wirkstoffhaltigen Schicht trennt, die sog. Isolierschicht. Die Isolierschicht wiederum, bzw. das Kernmaterial in Abwesenheit einer Isolierschicht, ist von der ebenfalls kugelschalenförmigen Wirkstoffschicht umgeben, die ihrerseits von einem Überzug umschlossen sein kann, der die Abriebfestigkeit und die Lagerstabilität der Pellets erhöht.

Ein Vorteil einer Darreichungsform in Form von Pellets ist die Möglichkeit der individuellen Dosierung, was etwa für die Medikation von Kindern von Bedeutung ist.

Als Kernmaterial werden eine pharmazeutisch akzeptable Base mit einer Wasserlöslichkeit von größer als 1 g / 250 ml bei 20° C, wie z.B. NaOH, KOH, $Ca(OH)_2$, $Na_2CO_3$, Ammoniak, Diethanolamin, Meglumin, Lysin, Arginin, Ethanolamin, Piperazin, Trometamol oder Triethanolamin, oder Mischungen aus solchen Basen verwendet, der gegebenenfalls ein geringer Anteil von 1 bis 10 Gew.-%, bevorzugt 3 bis 6 Gew.-% eines geeignete Bindemittels zugesetzt wird. Die Verwendung eines Bindemittels ist z.B. dann erforderlich, wenn das Kernmaterial in einem Kessel-aufbauverfahren hergestellt wird. Bei Verwendung eines Extrusions- oder Sphäronisierungs-Verfahrens benötigt man an Stelle von Bindemitteln andere technologische Hilfsstoffe, beispielsweise mikrokristalline Cellulose und gegebenenfalls ebenfalls Bindemittel um die mechanische Stabilität zu erhöhen. Es ist auch denkbar, reine (100 %-ige) Base als Startmaterial zu verwenden, wenn man diese in hinreichend enger Korngrößenverteilung beschaffen kann. Als pharmazeutisch akzeptable Base werden bevorzugt NaOH, KOH, $Ca(OH)_2$, Ammoniak, Diethanolamin, Meglumin, Lysin, Arginin oder Trometamol eingesetzt; besonders bevorzugt sind Meglumin, Lysin, Arginin und Trometamol oder deren Mischungen, ganz besonders bevorzugt ist Meglumin. Als Bindemittel können Gummi arabicum oder ein partial- oder vollsynthetisches Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus *N*-Vinylpyrrolidon und Vinylacetat, oder Kombinationen dieser Polymere verwendet werden; bevorzugt ist Gummi arabicum. Das sphärische Kernmaterial hat vorzugsweise einen mittleren Durchmesser von 0,4 - 1,5 mm. Der Gehalt der pharmazeutisch akzeptablen anorganischen oder organischen Base liegt üblicherweise zwischen 30 und 100 % im Kernmaterial. Dieses basehaltige spärische Kernmaterial wird auch als Starterpellets oder kurz "Starter" (z.B. Meglumin-Starter, Lysinstarter) bezeichnet.

Zur Erhöhung der Lagerstabilität des Fertigproduktes ist es bei Wirkstoffen, die in Gegenwart von Basen instabil sind, vorteilhaft, das Kernmaterial vor dem Auftrag des Wirkstoffes mit einer Isolierschicht basierend auf einem wasserlöslichen, pharmazeutisch akzeptablen Polymer zu beschichten. Als solches wasserlösliches Polymer kommen beispielsweise Gummi arabicum oder ein partial- oder vollsynthetisches Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere in Frage. Bevorzugt wird Gummi arabicum oder eine Hydroxypropylmethylcellulose verwendet. Gegebenenfalls kann die Beschichtung mit dem wasserlöslichen, pharmazeutisch akzeptablen Polymer unter Zusatz geeigneter Weichmacher, Trennmittel und Pigmente erfolgen, wie beispielsweise Triethylcitrat, Tributylcitrat, Triacetin, Polyethylenglykole (Weichmacher), Talkum, Kieselsäure (Trennmittel), Titandioxid oder Eisenoxidpigmente (Pigmente).

Die Wirkstoffschicht enthält den Wirkstoff sowie Binde- und gegebenenfalls Trennmittel. Als Bindemittel können beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus *N*-Vinylpyrrolidon und Vinylacetat oder Kombinationen dieser Polymere verwendet werden. Vorzugsweise wird Hydroxypropylcellulose oder Copolymerisate aus *N*-Vinylpyrrolidon und Vinylacetat eingesetzt. Der Zusatz von Trennmitteln wie z.B. Talkum, Magnesiumstearat oder Kieselsäure dient dazu, ein Zusammenwachsen der Partikel während des Prozesses zu verhindern. Der bevorzugte Wirkstoffgehalt beträgt maximal 60 %, vorzugsweise maximal 50 % der pharmazeutischen Zusammensetzung.

Die optionale äußerste Schicht, die zur Verminderung eines erhöhten Abriebs bei der Abfüllung in Kapseln und/oder zur Erhöhung der Lagerstabilität dient, besteht aus pharmazeutisch üblichen Filmbildnern, Weichmachern und gegebenenfalls Pigmenten. Als Filmbildner können beispielsweise Hydroxypropylcellulose, Hydroxypropylmethylcellulose, Methylcellulose, Polymerisate und Copolymerisate der Acryl- und Methacrylsäure und deren Estern, oder Kombinationen dieser Polymere verwendet werden. Als Weichmacher kommen unter anderem Triethylcitrat, Tributylcitrat, Triacetin oder Polyethylenglykole in Frage. Als Pigmente können z.B. Titandioxid oder Eisenoxidpigmente eingesetzt werden. Bevorzugt besteht der äußere Überzug aus Hydroxypropylmethylcellulose und / oder Methylcellulose, gegebenenfalls unter Zusatz von Polyethylenglykolen als Weichmacher.

Die Pellets können nach dem im folgenden beschriebenen Verfahren hergestellt werden:

Das basehaltige Kernmaterial besteht entweder aus Kristallen der jeweils zur Verwendung kommenden Base oder vorteilhafter aus annähernd sphärischen Partikeln in der gewünschten Größe mit einem hohen Gehalt an Base, die mittels Verfahren hergestellt werden können, die in der pharmazeutischen Technologie bekannt und etabliert sind. In Frage kommen insbesondere der Aufbau des Kernmaterials in Kesselverfahren, auf Pelletiertellern, oder mittels Extrusion / Sphäronisierung. Anschließend kann das so erhaltenen Kernmaterial durch Sieben in Fraktionen mit dem gewünschten Durchmesser geteilt werden. Geeignetes Kernmaterial hat einen mittleren Durchmesser von 0,4 bis 1,5 mm, bevorzugt von 0,6 bis 0,8 mm.

Auf dieses basehaltige Kernmaterial wird zunächst die Isolierschicht aufgetragen. Dies kann durch übliche Verfahren, z.B. durch Auftragen einer wäßrigen Dispersion des wasserlöslichen, pharmazeutisch akzeptablen Polymers gegebenenfalls unter Zusatz von Weichmachern, Trennmitteln und / oder Pigmenten in der Wirbelschicht, in Dragierkesseln oder in üblichen Filmcoatinganlagen, geschehen. Falls erforderlich, kann anschließend erneut gesiebt werden.

Daran anschließend wird der Wirkstoff aus einer bindemittel- und ggf. trennmittelhaltigen Dispersion aufgetragen. Das flüchtige Dispersionsmittel wird während des Prozesses und / oder daran anschließend durch Trocknen entfernt. Als Bindemittel in der Dispersion kann beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus *N*-Vinylpyrrolidon und Vinylacetat oder Kombinationen dieser Polymere verwendet werden. Vorzugsweise wird Hydroxypropylcellulose oder Copolymerisate aus *N*-Vinylpyrrolidon und Vinylacetat eingesetzt. Als Trennmittel eignen sich z.B. Talkum, Magnesiumstarat oder Kieselsäure; vorzugsweise wird Talkum verwendet. Als Dispersionsmittel kommen beispielsweise Wasser, Ethanol, 2-Propanol, Aceton oder Mischungen dieser Lösungsmittel miteinander in Frage. Der Wirkstoffauftrag auf das Kernmaterial kann mittels in der pharmazeutischen Technologie bekannter und etablierter Verfahren z.B. in Dragierkesseln, konventionellen Filmcoatinganlagen oder in der Wirbelschicht erfolgen. Anschließend kann erneut ein Siebprozess durchgeführt werden.

Zur Verminderung eines erhöhten Abriebs bei der Abfüllung in Kapseln oder zur Erhöhung der Lagerstabilität kann das System abschließend noch mit einer Schicht aus pharmazeutisch üblichen Filmbildnern, Weichmachern und ggf. Pigmenten überzogen werden. Dies kann mit Hilfe von üblichen Verfahren erfolgen, wie sie bereits bei der Beschreibung des Aufbringens der Isolierschicht erwähnt wurden.

Bei Verwendung von Kernmaterial mit einem mittleren Durchmesser von 0,4 - 1,5 mm werden durch das oben beschriebene Verfahren wirkstoffhaltige Pellets erhalten, die anschließend beispielsweise in Hartkapseln abgefüllt werden können. Dazu wird eine der Dosierung entsprechende Vielzahl dieser Einheiten auf Standard-Kapselfüllmaschinen in Hartkapseln gefüllt. Als geeignete Hartkapseln kommen beispielsweise Hartgelatinekapseln oder Hartkapseln aus Hydroxypropylmethylcellulose (HPMC) in Frage. Der bevorzugte Wirkstoffgehalt der pharmazeutischen Zusammensetzung beträgt maximal 60 %, vorzugsweise maximal 50 %.

Soweit nicht anders vermerkt, handelt es sich bei den Prozentangaben stets um Gewichtsprozente. Angaben zum Wirkstoffgehalt beziehen sich, soweit nicht anders angegeben, jeweils auf die Wirkstoffsäure (und nicht auf ein bestimmtes Salz).

Die Menge an Wirkstoff pro Kapsel wird vorzugsweise so gewählt, daß zur Erzielung der gewünschten Wirkung die Einnahme von 1 bis 2 Kapseln täglich ausreicht. Für Repaglinide kommen beispielsweise tägliche Gaben von 0,2 mg bis 5 mg, bevorzugt Kapseln enthaltend 0,5 mg, 1,0 mg oder 2,0 mg in Frage.

Für Telmisartan kommen beispielsweise tägliche Gaben von 10 mg bis 150 mg, bevorzugt Kapseln enthaltend 20 mg, 40 mg oder 80 mg in Frage.

Für Meloxicam kommen beispielsweise tägliche Gaben von 4 mg bis 30 mg, bevorzugt Kapseln enthaltend 7,5 mg oder 15,0 mg in Frage.

Für DT-TX 30 kommen beispielsweise tägliche Gaben von 50 mg bis 300 mg, bevorzugt Kapseln enthaltend 100 mg oder 200 mg in Frage.

Für Gliquidone kommen beispielsweise tägliche Gaben von 10 mg bis 50 mg, bevorzugt Kapseln enthaltend 30 mg in Frage.

Für Glibenclamide kommen beispielsweise tägliche Gaben von 1,9 mg bis 5,0 mg, bevorzugt Kapseln enthaltend 3,5 mg in Frage.

Das bevorzugte Base-Wirkstoffverhältnis beträgt ca. 1:1 bis ca. 20:1. Die theoretische Untergrenze, bei der das System noch funktionieren kann, liegt bei 1 Equivalent Base pro Mol des Wirkstoffs. Die Obergrenze von ca. 20:1 (Base zu Wirkstoff), wird durch die Größe der Darreichungsform bei den gewünschten Dosierungen bestimmt (Anzahl der Pellets pro Kapsel).

In der Qualitätskontrolle werden in vitro Freigaben mit USP Methoden gemessen. Hierbei wird die Arzneiform in einem Volumen von 900 ml freigesetzt und der pH so gewählt, daß "Sink-Conditions" bestehen, d.h. die vollständige Wirkstoffdosis ist in diesen 900 ml löslich. Diese in vitro Methodik kann in den meisten Fällen nicht prädiktiv für die Resorption beim Menschen sein, da dieser Arzneimittel meist mit ca. 200 ml Flüssigkeit einnimmt und bei anazidem Magen die Löslichkeit in vielen Fällen nur für einen Bruchteil der Dosis ausreicht. Anazider Magen kommt bei älteren Patienten in einer Häufigkeit von ca 25 % der Bevölkerung vor, häufig wird er auch durch Comedikation mit H2-Blockern oder Protonenpumpeninhibitoren hervorgerufen.

Es wurde daher im Rahmen der Erfindung eine empirische Testmethodik entwickelt, die eine bessere Korrelation zur in vivo Performance am Mensch aufweist. Hierzu wird eine Arzneiform, die die höchste am Menschen angewendete Dosis enthält, in einem Volumen von 200 ml (dies entspricht der Humananwendung) in Puffer bei einem pH Wert mit niedriger Löslichkeit des Wirkstoffs im physiologisch sinnvollen Bereich, d.h. zwischen pH 1 - 7 freigesetzt. Da sich mit dieser Methode die Resorbierbarkeit auch bei anazidem Magen-pH gut vorhersagen läßt, ist sie für die Optimierung von Arzneiformen gut geeignet. Um aus vielen Rezepturbeispielen die jeweils günstigste Formulierung zu identifizieren, kann die maximale Freigabe und/oder die Fläche unter dem Kurvenverlauf (AUC) vom Zeitpunkt 0 bis zum Endpunkt der Freigabe als relevante Kenngrößen verwendet werden.

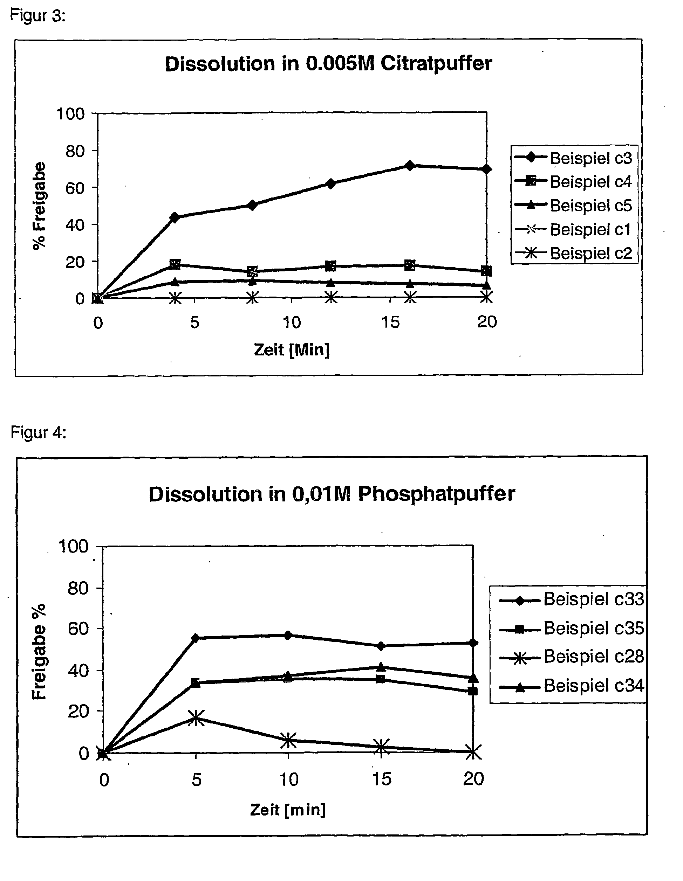

Dies wird am Beispiel des Vergleichs der Formulierungsbeispiele c1 - c2 (Referenz auf Neutralstarter) und c3 - c5 (erfindungsgemäßes Beispiel mit Megluminstarter) deutlich (Figur 3 und Tabelle 1). Unter "Neutralstarter", "Neutralkern" bzw. "Neutralpellet" sind jeweils handelsübliche Pellets aus Saccharose oder mikrokristalliner Cellulose zu verstehen.

Tabelle 1a zeigt die in vitro Freigaben und die Kenndaten Fläche unter der Freigabekurve (AUC) und maximale Freigabe der erfindungsgemäßen Beispiele c3 - c5 (Wirkstoff: Telmisartan) im Vergleich zu den Referenzformen der Beispiele c1 und c2 mit sogar noch geringerem Wirkstoffgehalt in 0.005 Mol Citratpuffer pH 5.0

Tabelle 1a: Vergleich der in vitro Freigaben von Pellets auf Basestartern (Beispiele c3 - c5) und Neutralstartern (Beispiele c1- c2)

| Beispiel | Wirkstoffgehalt | in vitro Freigabe nach ... Minuten | | | | | | AUC | maximale Freigabe |
|---|---|---|---|---|---|---|---|---|---|
| | | 0 | 4 | 8 | 12 | 16 | 20 | | |
| c1 | 2.6 | 0 | 0 | 0 | 0 | 0 | 0 | 0 | 0 |
| c2 | 16.8 | 0 | 0 | 0 | 0 | 0 | 0 | 0 | 0 |
| c3 | 8.9 | 0 | 44 | 50 | 62 | 72 | 69 | 1046 | 72 |
| c4 | 23.2 | 0 | 18 | 14 | 17 | 17 | 14 | 293 | 18 |
| c5 | 29.4 | 0 | 9 | 9 | 8 | 8 | 6 | 147 | 9 |

Weitere Beispiele sind die Vergleiche der Formulierungsbeispiele c28 (Referenz auf Neutralstarter) mit c31, c32 (erfindungsgemäßes Beispiel mit Megluminstarter) bzw. c33- c35 (erfindungsgemäßes Beispiel mit Argininstarter) sowie die Vergleiche der Formulierungsbeispiele c43 und c45 (Referenz auf Neutralstarter) mit c46 und c47 (erfindungsgemäßes Beispiel mit Megluminstarter) und c49 und c49a (erfindungsgemäßes Beispiel mit Argininstarter).

Tabelle 1b zeigt die in vitro Freigaben und die Kenndaten Fläche unter der Freigabekurve (AUC) und maximale Freigabe der erfindungsgemäßen Beispiele c31, c32, c33, c34 und c35 (Wirkstoff: Meloxicam) im Vergleich zu der Referenzform des Beispiels c28 in 0.01 Mol Phosphatpuffer pH 5.0

Tabelle 1b: Vergleich der in vitro Freigaben von Pellets auf Basestartern (Beispiele c31, c32, c33, c34, c35) und Neutralstartern (Beispiel c28)

| Beispiel | Wirkstoffgehalt | in vitro Freigabe nach ... Minuten | | | | | AUC | maximale Freigabe |
|---|---|---|---|---|---|---|---|---|
| | | 0 | 5 | 10 | 15 | 20 | | |
| c28 | 16.5 | 0 | 17 | 6 | 3 | 0 | 126 | 17 |
| c31 | 16.8 | 0 | 71 | 83 | 87 | 88 | 1426 | 88 |
| c32 | 26.1 | 0 | 58 | 61 | 62 | 52 | 1034 | 62 |
| c33 | 17.1 | 0 | 55 | 57 | 52 | 53 | 951 | 57 |
| c34 | 28.6 | 0 | 34 | 37 | 41 | 36 | 652 | 41 |
| c35 | 33.3 | 0 | 34 | 36 | 35 | 29 | 596 | 36 |

Tabelle 1c zeigt die in vitro Freigaben und die Kenndaten Fläche unter der Freigabekurve (AUC) und maximale Freigabe der erfindungsgemäßen Beispiele c46, c47, c49 und c49a (Wirkstoff: DT-TX 30) im Vergleich zu Referenzformen der Beispiele c43 und c45 in 0.025 Mol Phosphatpuffer pH 6.0

Tabelle 1c: Vergleich der in vitro Freigaben von Pellets auf Basestartern (Beispiele c46, c47, c49 und c49a und Neutralstartern (Beispiele c43, c45)

| Beispiel | Wirkstoffgehalt | in vitro Freigabe nach ... Minuten | | | | | AUC | maximale Freigabe |
|---|---|---|---|---|---|---|---|---|
| | | 0 | 4 | 8 | 12 | 16 | 20 | | |
| c43 | 15.8 | 0 | 7 | 6 | 5 | 0 | 0 | 74 | 7 |
| c45 | 31.2 | 0 | 7 | 8 | 0 | 0 | 0 | 58 | 8 |
| c46 | 16.3 | 0 | 19 | 51 | 55 | 56 | 58 | 845 | 58 |
| c47 | 25.7 | 0 | 8 | 35 | 38 | 39 | 39 | 558 | 39 |
| c49 | 26.7 | 0 | 19 | 29 | 27 | 27 | 27 | 463 | 29 |
| c49a | 19.5 | 0 | 30 | 32 | 30 | 35 | 36 | 578 | 36 |

Interpretation der Ergebnisse:

Alle erfindungsgemäßen Beispiele sind den Referenzformulierung deutlich überlegen, da die Referenzformen keine oder nur geringe messbaren Freigaben erreichen.

Mit zunehmendem Wirkstoffgehalt nimmt die in vitro Freigabe ab, da bei gleicher Wirkstoffdosis die Basemenge geringer wird.

Alle Basen und sonstigen Hilfsstoffe sind generell geeignet, zeigen aber bei vergleichbaren Wirkstoffkonzentration etwas unterschiedliches Freigabeverhalten. Als besonders geeignet im Sinne der Erfindung erwiesen sich die Beispiele c3, c4, c16, c21, c24, c25, c31, c32, c33, c46, c47, c49 und c49a.

Ein weitere Erfindungsgegenstand sind basehaltige Darreichungsformen für amphotere Wirkstoffe, wie beispielsweise Telmisartan.

Besonders überraschend ist die Überlegenheit der erfindungsgemäßen Formen bei amphoteren, d.h. sauer und basisch löslichen Wirkstoffen, wenn man die in vitro Freigaben der erfindungsgemäßen Pellets auf Basestartern mit Pellets vergleicht, bei denen der Wirkstoff auf säurehaltige Kerne aufgetragen wurde. Bei ähnlicher Wirkstoffbeladung mit einem amphoteren Wirkstoff ist die Freigabe der basehaltigen Pellets deutlich besser als die der säurehaltigen Pellets wie der Vergleich der in vitro Freigaben der Beispiele c10, c13, c14 und c15 (Freigaben s. Tabelle 2 und Figur 1) zeigt.

Tabelle 2: Vergleich der in vitro Freigaben und der Kenndaten Fläche unter der Freigabekurve (AUC) und maximale Freigabe von Pellets auf Basestartern (Beispiele c3 - c5) und Säurestartern (Beispiele c13 - c15 enthalten Weinsäurestarter und Beispiel c10 enthält Bernsteinsäurestarter)

| Beispiel | Wirkstoffgehalt | in vitro Freigabe nach ... Minuten | | | | | | AUC | maximale Freigabe |
|---|---|---|---|---|---|---|---|---|---|
| | | 0 | 4 | 8 | 12 | 16 | 20 | | |
| c3 | 8.9 | 0 | 44 | 50 | 62 | 72 | 69 | 1046 | 72 |
| c4 | 23.2 | 0 | 18 | 14 | 17 | 17 | 14 | 293 | 18 |
| c5 | 29.4 | 0 | 9 | 9 | 8 | 8 | 6 | 147 | 9 |
| c13 | 3.4 | 0 | 16 | 17 | 17 | 17 | 18 | 302 | 18 |
| c14 | 9.5 | 0 | 7 | 8 | 8 | 7 | 8 | 134 | 8 |
| c15 | 17.4 | 0 | 4 | 4 | 3 | 3 | 3 | 63 | 4 |
| c10 | 19.7 | 0 | 0 | 0 | 0 | 0 | 0 | 0 | 0 |

Die Daten zeigen dass Beispiel c3 mit ca 9% Wirkstoffgehalt eine maximale Freigabe von 72 % Freigabe erreicht, während Beispiel c13 mit ebenfalls ca 9% Wirkstoffgehalt eine maximale Freigabe von nur 8 % Freigabe erreicht, d.h. die maximale Freigabe mit Basestarter liegt um den Faktor 9 höher, hinsichtlich AUC wird der Faktor 8 erreicht. Beispiel c4 mit ca 23% Wirkstoffgehalt erreicht 18 % Freigabe während Beispiel c15 mit nur 17,4% Wirkstoffgehalt nur 4% Freigabe erreicht, d.h. die maximale Freigabe und auch der AUC-Wert liegt um den Faktor 5 höher. Beispiel c5 mit 29 % Wirkstoffgehalt erreicht noch 9 % Freigabe und ist somit vergleichbar mit Beispiel c14 mit nur 9,5 % Wirkstoffgehalt. Dies bedeutet dass man z.B. für 1 Kapsel mit 80 mg Wirkstoff für Beispiel c5 nur 235 mg Pellets benötigt, die sehr gut in die Kapselgröße 3 (ca 6*16 mm) abfüllbar sind, während für 1 Kapsel mit 80 mg Wirkstoff für Beispiel c14 aber 762 mg Pellets benötigt werden, die nur noch in 2 Kapseln der Kapselgröße 1 (ca 8*24 mm) abfüllbar sind, d.h. die Einnahme ist für den Patienten deutlich erschwert. Beispiele c15 mit 17 % Wirkstoffgehalt erreicht nur 4 % maximale Freigabe und Beispiel c10 (Bernsteinsäurestarter mit 20 % Wirkstoffgehalt) gibt den Wirkstoff überhaupt nicht mehr frei.

Legende zu den Figuren:

Figur 1 zeigt die in vitro Freigaben der erfindungsgemäßen Beispiele c3 - c5 im Vergleich zur Formulierungen auf Weinsäurestartern (Beispiele c13 - c15) und auf Bernsteinsäurestarter (c10) mit unterschiedlichen Wirkstoffgehalten in 0.005 Mol Citratpuffer pH 5.0.

Figur 2 zeigt den schematischen Aufbau einer erfindungsgemäßen pharmazeutischen Zusammensetzung in Form einer Schnittdarstellung eines Pellets.

Figur 3 zeigt die in vitro Freigaben der erfindungsgemäßen Beispiele c3 - c5 im Vergleich zu den Referenzformulierungen c1 - c2 mit unterschiedlichen Wirkstoffgehalten in 0.005 Mol Citratpuffer pH 5.0.

Figur 4 zeigt die in vitro Freigaben der erfindungsgemäßen Beispiele c33 - c35 im Vergleich zu der Referenzformulierung c28 mit unterschiedlichen Wirkstoffgehalten in 0.01 Mol Phosphatpuffer pH 5.0.

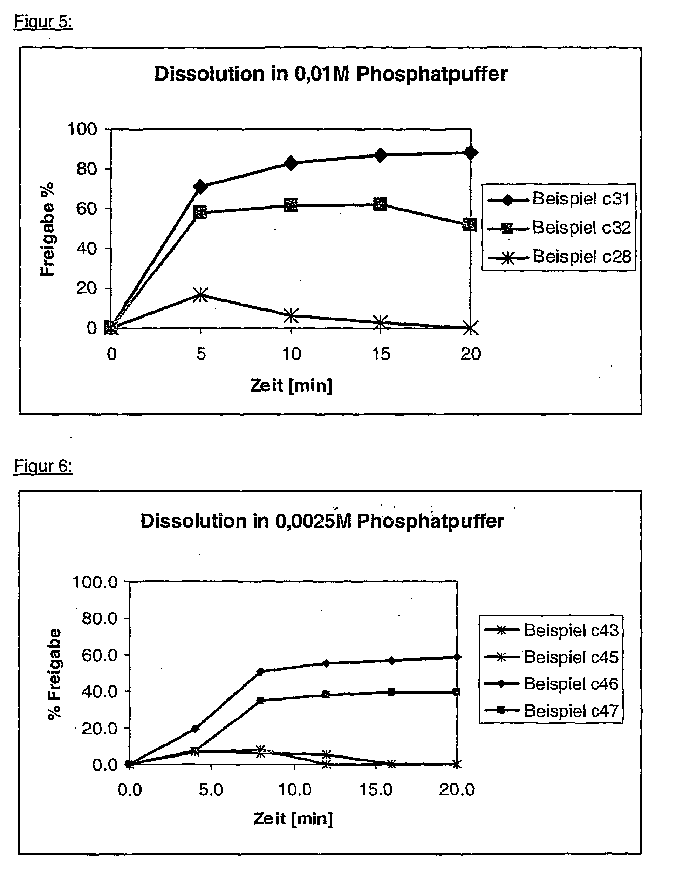

Figur 5 zeigt die in vitro Freigaben der erfindungsgemäßen Beispiele c31 und c32 im Vergleich zu der Referenzformulierung c28 mit unterschiedlichen Wirkstoffgehalten in 0.01 Mol Phosphatpuffer pH 5.0.

Figur 6 zeigt die in vitro Freigaben der erfindungsgemäßen Beispiele c46 und c47 im Vergleich zu den Referenzformulierungen c43 und c45 mit unterschiedlichen Wirkstoffgehalten in 0.0025 Mol Phosphatpuffer pH 6.0.

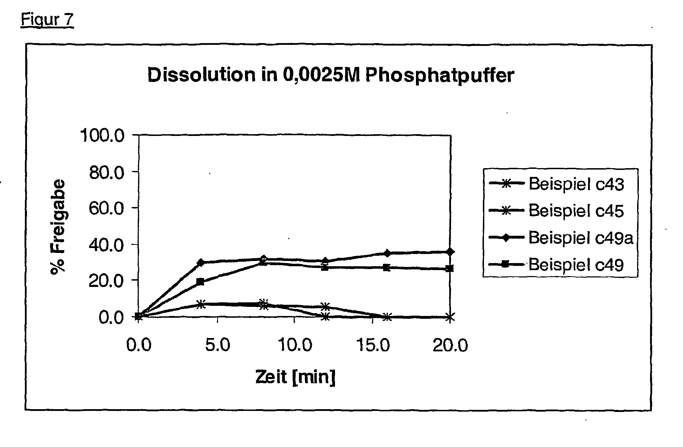

Figur 7 zeigt die in vitro Freigaben der erfindungsgemäßen Beispiele c49 und c49a im Vergleich zu den Referenzformulierungen c43 und c45 mit unterschiedlichen Wirkstoffgehalten in 0.0025 Mol Phosphatpuffer pH 6.0.

Die nachfolgenden Beispiele sollen die Erfindung näher erläutern:

**Beispiele:**

Die Herstellung der nachfolgenden Beispiele erfolgt generell in 5 Arbeitsschritten:

a: *Aufbau von basehaltigem Kernmaterial*
b: *Isolierung des basehaltigen Kernmaterials*
c: *Aufbau der Wirkstoffschicht*
d: *Isolierung des Pellets bestehend aus Basehaltigem Kern*
e: *Abfüllung in Kapseln*

Schritt b ist bei Inkompatibilität zwischen Base und Wirkstoffschicht zwingend notwendig, ansonsten kann auf diesen Schritt zur Vereinfachung der Herstelltechnologie verzichtet werden. Schritt d ist notwendig, wenn die mechanische Stabilität der Wirkstoffschicht nicht für vollständige Auflösung des Wirkstoffs ausreicht.

Die in den Beispielen verwendeten und nicht gesondert gekennzeichneten Handelsnamen stehen für folgende Substanzen:

| | |
|---|---|
| Kollidon K25 | Povidone (Polyvinylpyrrolidon) |
| Avicel PH101 | mikrokristalline Cellulose |
| Klucel | Hydroxypropylcellulose |

d.h. die betreffenden Beispiele sind so zu lesen, dass man Povidone, z.B. Kollidon K25 verwendet.

a: *Beispiele für den Aufbau von Basehaltigem Kernmaterial*

Beispiel a1: Aufbau von megluminhaltigem Kernmaterial mit Bindemittel

Zusammensetzung:

| | |
|---|---|
| Kollidon K25 | 3 Gewichtsteile |
| Avicel PH101 | 20 Gewichtsteile |
| Meglumin | 77 Gewichtsteile |

77 Gewichtsteile Meglumin, 20 Gewichtsteile Avicel PH 101 und 3 Gewichtsteile Kollidon K25 werden in einem Rhönradmischer 15 Minuten lang gemischt. Dann wird die Pulvermischung in einen Doppelschnecken-Dosierer überführt. Diese Mischung wird mit einer Geschwindigkeit von ca 1 kg/h zusammen mit Wasser, das mit einer ProMinent Dosierpumpe zugegeben wird in einen Doppelschnecken-Extruder Typ Werner & Pfleiderer 37/18D (oder einen anderen geeigneten Extrudertyp) eingebracht. Die Wasser wird automatisch so geregelt, daß im Extruder ein Solldrehmoment ca. 19% erzeugt wird. Die Extrusion erfolgt über eine Düsenplatte mit Bohrungen von 8 mm Durchmesser.
Die Ausrundung der Extrusionsstränge zu Pellets erfolgt in einem WyPro-Sphäromat, wobei ca. 3 Minuten bei ca. 850 RPM gerundet wurde.
Trocknung der Pellets bei 80°C ca. 1,5 h im GPCG1 Wirbelschichttrockner.

Das Kernmaterial wird über eine Taumelsiebmaschine mit unterschiedlichen Siebböden mit nominalen Maschenweiten von 0,71 bis 1,25 mm fraktioniert. Die jeweils geeigneten Gutfraktionen zwischen 0,71 und 0,90 bzw. 0,90 und 1,12 mm werden in den weiteren Prozessen verwendet.

Beispiel a2: Aufbau von megluminhaltigem Kernmaterial ohne Bindemittel

Zusammensetzung:

| | |
|---|---|
| Avicel PH101 | 40 Gewichtsteile |

(fortgesetzt)

| | |
|---|---|
| Meglumin | 60 Gewichtsteile |

60 Gewichtsteile Meglumin und 40 Gewichtsteile Avicel PH 101 werden in einem Rhönradmischer 15 Minuten lang gemischt. Dann wird die Pulvermischung in einen Doppelschnecken-Dosierer überführt. Diese Mischung wird mit einer Geschwindigkeit von ca 1 kg/h zusammen mit Wasser, das mit einer ProMinent Dosierpumpe zugegeben wird in einen Doppelschnecken-Extruder Typ Werner & Pfleiderer 37/18D (oder einen anderen geeigneten Extrudertyp) eingebracht. Die Wasserdosierung wird automatisch so geregelt, daß im Extruder ein Solldrehmoment ca. 19% erzeugt wird. Die Extrusion erfolgt über eine Düsenplatte mit Bohrungen von 8 mm Durchmesser.

Die Ausrundung der Extrusionsstränge zu Pellets erfolgt in einem WyPro-Sphäromat, wobei ca. 3 Minuten bei ca. 850 RPM gerundet wurde.

Trocknung der Pellets bei 80°C ca. 1,5h im GPCG1 Wirbelschichttrockner.

Das Kernmaterial wird über eine Taumelsiebmaschine mit unterschiedlichen Siebböden mit nominalen Maschenweiten von 0,71 bis 1,25 mm fraktioniert. Die jeweils geeigneten Gutfraktionen zwischen 0,71 und 0,90 bzw. 0,90 und 1,12 mm werden in den weiteren Prozessen verwendet.

Beispiel a3: Aufbau von argininhaltigem Kernmaterial

Zusammensetzung:

| | |
|---|---|
| Avicel PH101 | 40 Gewichtsteile |
| Arginin | 60 Gewichtsteile |

Herstellung analog Beispiel a2

Beispiel a4: Aufbau von trometamolhaltigem Kernmaterial

Zusammensetzung:

| | |
|---|---|
| Avicel PH101 | 40 Gewichtsteile |
| Trometamol | 60 Gewichtsteile |

Herstellung analog Beispiel a2

Beispiel a5: Aufbau von piperazinhaltigem Kernmaterial

Zusammensetzung:

| | |
|---|---|
| Avicel PH101 | 40 Gewichtsteile |
| Piperazin | 60 Gewichtsteile |

Herstellung analog Beispiel a2

Beispiel a6: Aufbau von natiumhydroxidhaltigem Kernmaterial

Zusammensetzung:

| | |
|---|---|
| Avicel PH101 | 30 Gewichtsteile |
| Natriumhydroxid | 70 Gewichtsteile |

Die Herstellung erfolgt durch Auflösen von Natriumhydroxid in Wasser, anschließend wird Avicel PH101 zugegeben. Die weitere Verarbeitung erfolgt analog Beispiel a2

Beispiel a7: Aufbau von kaliumhydroxidhaltigem Kernmaterial

Zusammensetzung:

| | |
|---|---|
| Avicel PH101 | 40 Gewichtsteile |
| Kaliumhydroxid | 60 Gewichtsteile |

Herstellung analog Beispiel a6

Beispiel a8: Aufbau von calciumhydroxidhaltigem Kernmaterial

Zusammensetzung:

| | |
|---|---|
| Avicel PH101 | 70 Gewichtsteile |
| Calciumhydroxid | 30 Gewichtsteile |

Herstellung analog Beispiel a6

Beispiel a9: Aufbau von natiumhydroxid- und mealuminhaltigem Kernmaterial

Zusammensetzung:

| | |
|---|---|
| Avicel PH101 | 30 Gewichtsteile |
| Natriumhydroxid | 20 Gewichtsteile |
| Meglumin | 50 Gewichtsteile |

Die Herstellung erfolgt durch Auflösen von Natriumhydroxid in Wasser, anschließend' werden Avicel PH101 und Meglumin zugegeben. Die weitere Verarbeitung erfolgt analog Beispiel a2

*b: Beispiel für die Isolierung des basehaltigen Kernmaterials*

Zusammensetzung:

| | |
|---|---|
| Basehaltiges Kernmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile basehaltiges Kernmaterial bei einer Zulufttemperatur von 35° - 40° C mit der Gummi arabicum-Talkum - Dispersion im Unterbettsprühverfahren besprüht.

Das isolierte basehaltige Kernmaterial wird anschließend im Umlufttrockenschrank bei 40° C über 8 Stunden nachgetrocknet.

Zur Entfernung von Agglomeraten wird das getrocknete isolierte basehaltige Kernmaterial mit einem Sieb mit der nominalen Maschenweite 1,0 mm gesiebt. Die Gutfraktion (Korngröße kleiner als 1 mm) wird weiterverarbeitet.

*c: Beispiele für den Aufbau der Wirkstoffschicht*

Der Aufbau der Wirkstoffschicht erfolgt generell in immer gleicher Weise, wobei aber Wirkstoffart und Menge, Bindemittelart und Menge, Talkummenge und Isopropanol bzw. Äthanolmenge variiert werden. Die Herstellung wird deshalb nur für Beispiel c9 beschrieben, die jeweiligen Zusammensetzungen werden für jeden Wirkstoff in Tabellenform dargestellt.

Herstellung von Beispiel c9:

Zusammensetzung:

| | |
|---|---|
| Isoliertes megluminhaltiges Kernmaterial | 12 Gewichtsteile |
| Hydroxypropylcellulose | 2.5 Gewichtsteile |
| Talkum | 5 Gewichtsteile |
| Wirkstoff (z.B. Telmisartan) | 10 Gewichtsteile |

In 255 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 12 Gewichtanteile megiuminhaltiges-Kernmaterial bei einer Zulufttemperatur von 20° - 30° C mit der wirkstoffhaltigen ; Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße kleiner als 1,25 mm) wird weiterverarbeitet.

Weitere Beispiels für Schritt c s. u.

*d: Beispiel für die Isolierung der wirkstoffhaltigen Pellets*

Zusammensetzung:

| | |
|---|---|
| Wirkstoffhaltige Pellets | 23 Gewichtsteile |
| Gummi arabicum . | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile wirkstoffhaltige Pellets bei einer Zulufttemperatur von 35° - 40° C mit der Gummi arabicum-Talkum - Dispersion im Unterbettsprühverfahren besprüht.

Das isolierte megluminhaltige Kernmaterial wird anschließend im Umlufttrockenschrank bei 40° C über 8 Stunden nachgetrocknet.

Zur Entfernung von Agglomeraten werden die getrockneten wirkstoffhaltigen Pellets mit einem Sieb mit der nominalen Maschenweite 1,25 mm gesiebt. Die Gutfraktion (Korngröße kleiner als 1.25 mm) wird weiterverarbeitet.

*e) Abfüllung in Kapseln*

Eine jeweils der gewünschten Dosierung entsprechende Menge an wirkstoffhaltigen Pellets wird mittels einer Kapselfüllmaschine in Hartkapseln, z.B. in Hartgelatinekapseln, einer geeigneten Größe abgefüllt.

**Weitere Zusammenseztungsbeispiele für Schritt c**

Die im folgenden angegebenen Zahlen bedeuten, soweit nicht anders angegeben, Gewichtsanteile. Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.
Beispielsweise können die 10-fachen Werte als Angaben in Gramm angesehen werden, also für Beispiel c1 200,0 g Neutralpellets, 5,4 g Telmisartan, 1,4 g Povidone K90, 2,7 g Talkum und 142,1 g Isopropanol.
Die Beispiele, die einen im Handel erhältlichen Neutralkern anstelle der erfindungsgemäßen basehaltigen Starterkerne enthalten, dienen jeweils als Referenzwerte für die in vitro Testung.

Telmisartanbeispiele

Die Beispiele c1 - c2 enthalten einen im Handel erhältlichen Neutralkern anstelle der erfindungsgemäßen basehaltigen Starterkerne. Diese Kerne dienen als Referenzwerte für die in vitro Testung (siehe Tabelle 1). Die Beispiele c10 - c15 enthalten säurehaltige Kerne anstelle der erfindungsgemäßen basehaltigen Starterkerne, um den nicht vorhersehbaren Vorteil der erfindungsgemäßen Basestartern gegenüber Säurestartern aufzuzeigen, die theoretisch mindestens gleich gute Auflösung zeigen sollten, da Telmisartan auch im Sauren gut löslich ist.

| Beispiel | c1 | c2 |
|---|---|---|
| Wirkstoffgehalt (Gew.-%): | 2.60 | 16.80 |
| Neutralpellets | 20.00 | 20.00 |
| Telmisartan | 0.54 | 4.76 |
| Povidone K 90 | 0.14 | 1.19 |
| Talkum | 0.27 | 2.38 |
| Isopropanol (nur zur Herstellung) | 14.21 | 124.36 |

| Beispiel | c3 | c4 | c5 | c6 | c7 | c8 | c9 |
|---|---|---|---|---|---|---|---|
| Wirkstoffgehalt (Gew.-%): | 8.9 | 23.2 | 29.4 | 33.7 | 10.6 | 14.5 | 33.7 |
| Megluminstarter | 12.00 | 12.00 | 12.00 | 12.00 | 12.00 | 12.00 | 12.00 |
| Telmisartan | 1.26 | 4.68 | 7.28 | 9.88 | 1.56 | 2.34 | 9.88 |
| Povidone K 90 | 0.32 | 1.17 | 1.82 | 2.47 | - | - | - |
| Talkum | 0.63 | 2.34 | 3.64 | 4.94 | 0.78 | 1.17 | 4.94 |
| Klucel | - | - | - | - | 0.39 | 0.59 | 2.47 |
| Isopropanol (nur zur Herstellung) | 32.94 | 122.27 | 190.19 | 258.12 | 40.37 | 60.55 | 255.65 |

| Beispiel | c10 | c11 | c12 |
|---|---|---|---|
| Wirkstoffgehalt (Gew.-%): | 19.70 | 20.70 | 32.10 |
| Bernsteinsäure-Starter | 12.00 | 12.00 | 12.00 |
| Telmisartan | 3.61 | 3.90 | 8.81 |
| Talkum | 1.81 | 1.95 | 4.41 |
| Klucel | 0.90 | 0.98 | 2.20 |
| Isopropanol (nur zur Herstellung) | 93.51 | 100.91 | 228.06 |

| Beispiel | c13 | c14 | c15 |
|---|---|---|---|
| Wirkstoffgehalt (Gew.-%): | 3.40 | 9.50 | 17.40 |
| Weinsäure-Starter | 20.00 | 20.00 | 20.00 |
| Telmisartan | 0.73 | 2.29 | 4.99 |
| Talkum | 0.36 | 1.14 | 2.50 |
| Klucel | 0.18 | 0.57 | 1.25 |
| Isopropanol (nur zur Herstellung) | 18.84 | 59.20 | 129.17 |

| Beispiel | c16 | c17 | c18 |
|---|---|---|---|
| Wirkstoffgehalt (Gew.-%): | 19.70 | 28.50 | 32.90 |
| Arginin-Starter | 12.00 | 12.00 | 12.00 |
| Telmisartan | 3.60 | 6.80 | 9.30 |
| Povidone K90 | 0.90 | 1.70 | 3.40 |
| Talkum | 1.80 | 3.40 | 4.65 |
| Isopropanol (nur zur Herstellung) | 93.60 | 176.80 | 241.80 |

| Beispiel | c19 | c20 |
|---|---|---|
| Wirkstoffgehalt (Gew.-%): | 17.10 | 26.20 |
| Trometamol-Starter | 12.00 | 12.00 |
| Telmisartan | 2.93 | 5.80 |
| Povidone K 90 | 0.73 | 1.45 |
| Talkum | 1.47 | 2.90 |
| Isopropanol (nur zur Herstellung) | 76.18 | 150.80 |

| Beispiel | c21 | c22 | c23 |
|---|---|---|---|
| Wirkstoffgehalt (Gew.-%): | 19.80 | 28.50 | 32.90 |
| Lysin-Starter | 12.00 | 12.00 | 12.00 |
| Telmisartan | 3.64 | 6.82 | 9.29 |
| Povidone K90 | 0.91 | 1.71 | 3.32 |
| Talkum | 1.82 | 3.41 | 4.65 |
| Isopropanol (nur zur Herstellung) | 94.64 | 177.32 | 241.54 |

| Beispiel | c24 | c25 |
|---|---|---|
| Wirkstoffgehalt (Gew.-%): | 18.20 | 25.80 |
| Natriumhydroxid-Starter | 12.00 | 12.00 |
| Telmisartan | 3.20 | 5.63 |
| Povidone K 90 | 0.80 | 1.41 |
| Talkum | 1.60 | 2.82 |
| Isopropanol (nur zur Herstellung) | 83.20 | 146.38 |

| Beispiel | c26 | c27 |
|---|---|---|
| Wirkstoffgehalt (Gew.-%): | 26.90 | 33.90 |
| Kaliumhydroxid-Starter | 12.00 | 12.00 |
| Telmisartan | 6.12 | 9.98 |
| Povidone K 90 | 1.53 | 2.50 |
| Talkum | 3.06 | 4.99 |
| Isopropanol (nur zur Herstellung) | 159.12 | 259.48 |

Meloxicambeispiele:

| Beispiel | c28 | c29 | c30 |
|---|---|---|---|
| Wirkstoffgehalt (Gew.-%): | 14.80 | 25.20 | 33.00 |
| Neutralpellets | 12.00 | 12.00 | 12.00 |
| Meloxicam | 2.40 | 5.42 | 9.40 |
| Povidone K90 | 0.60 | 1.36 | 2.35 |
| Talkum | 1.20 | 2.71 | 4.70 |
| Isopropanol (nur zur Herstellung) | 62.40 | 140.92 | 244.40 |

| Beispiel | c31 | c32 |
|---|---|---|
| Wirkstoffgehalt (Gew.-%): | 16.80 | 26.10 |
| Meglumin-Starter | 12.00 | 12.00 |
| Meloxicam | 2.85 | 5.78 |
| Povidone K 90 | 0.71 | 1.45 |
| Talkum | 1.43 | 2.89 |
| Isopropanol (nur zur Herstellung) | 74.10 | 150.28 |

| Beispiel | c33 | c34 | c35 |
|---|---|---|---|
| Wirkstoffgehalt (Gew.-%): | 17.10 | 28.60 | 33.30 |
| Arginin-Starter | 12.00 | 12.00 | 12.00 |
| Meloxicam | 2.93 | 6.87 | 9.58 |
| Povidone K90 | 0.73 | 1.72 | 2.40 |
| Talkum | 1.47 | 3.44 | 4.79 |
| Isopropanol (nur zur Herstellung) | 76.18 | 178.62 | 249.08 |

| Beispiel | c36 | c37 |
|---|---|---|
| Wirkstoffgehalt (Gew.-%): | 15.90 | 25.70 |
| Trometamol-Starter | 12.00 | 12.00 |
| Meloxicam | 2.64 | 5.61 |
| Povidone K 90 | 0.66 | 1.40 |
| Talkum | 1.32 | 2.81 |
| Isopropanol (nur zur Herstellung) | 68.64 | 145.86 |

| Beispiel | c38 | c39 | c40 |
|---|---|---|---|
| Wirkstoffgehalt (Gew.-%): | 16.10 | 26.50 | 36.60 |
| Lysin-Starter | 12.00 | 12.00 | 12.00 |
| Meloxicam | 2.70 | 5.94 | 11.30 |
| Povidone K90 | 0.68 | 1.49 | 2.83 |
| Talkum | 1.35 | 2.97 | 5.65 |
| Isopropanol (nur zur Herstellung) | 70.20 | 154.44 | 293.80 |

| Beispiel | c41 | c42 |
|---|---|---|
| Wirkstoffgehalt (Gew.-%): | 28.30 | 36.60 |
| Calciumhydroxid-Starter | 12.00 | 12.00 |
| Meloxicam | 6.75 | 12.21 |
| Povidone K 90 | 1.69 | 3.05 |
| Talkum | 3.38 | 6.11 |
| Isopropanol (nur zur Herstellung) | 175.50 | 317.46 |

DT-TX 30-Beispiele

| Beispiel | c43 | c44 | c45 |
|---|---|---|---|
| Wirkstoffgehalt (Gew.-%): | 15.80 | 22.20 | 31.20 |
| Neutralpellets | 12.00 | 12.00 | 12.00 |
| DT-TX 30 | 2.63 | 4.35 | 8.26 |
| Povidone K90 | 0.66 | 1.09 | 2.07 |
| Talkum | 1.32 | 2.18 | 4.13 |
| Isopropanol (nur zur Herstellung) | 68.38 | 113.10 | 214.76 |

| Beispiel | c46 | c47 | c48 |
|---|---|---|---|
| Wirkstoffgehalt (Gew.-%): | 16.30 | 25.70 | 33.60 |
| Meglumin-Starter | 12.00 | 12.00 | 12.00 |
| DT-TX 30 | 2.73 | 5.60 | 9.76 |
| Povidone K90 | 0.68 | 1.40 | 2.44 |
| Talkum | 1.37 | 2.80 | 4.88 |
| Isopropanol (nur zur Herstellung) | 70.98 | 145.60 | 253.76 |

| Beispiel | c49 | c49a | c50 |
|---|---|---|---|
| Wirkstoffgehalt (Gew.-%): | 26.70 | 19.50 | 34.20 |
| Arginin-Starter | 12.00 | 12.00 | 12.00 |
| DT-TX 30 | 6.00 | 4.00 | 10.20 |
| Povidone K 90 | 1.50 | 1.50 | 2.55 |
| Talkum | 3.00 | 3.00 | 5.10 |
| Isopropanol (nur zur Herstellung) | 156.00 | 156.00 | 265.20 |

| Beispiel | c51 | c52 | c53 |
|---|---|---|---|
| Wirkstoffgehalt (Gew.-%): | 15.70 | 26.00 | 37.60 |
| Lysin-Starter | 12.00 | 12.00 | 12.00 |
| DT-TX 30 | 2.59 | 5.74 | 13.20 |
| Povidone K90 | 0.65 | 1.44 | 3.30 |
| Talkum | 1.30 | 2.87 | 6.60 |
| Isopropanol (nur zur Herstellung) | 67.34 | 149.24 | 343.20 |

Gliguidonebeispiele

| Beispiel | c54 | c55 | c56 |
|---|---|---|---|
| Wirkstoffgehalt (Gew.-%): | 14.80 | 21.70 | 31.90 |
| Neutralpellets | 12.00 | 12.00 | 12.00 |
| Glilquidone | 2.40 | 4.21 | 8.67 |
| Povidone K90 | 0.60 | 1.05 | 2.17 |
| Talkum | 1.20 | 2.11 | 4.34 |
| Isopropanol (nur zur Herstellung) | 62.40 | 109.46 | 225.42 |

| Beispiel | c57 | c58 | c59 |
|---|---|---|---|
| Wirkstoffgehalt (Gew.-%): | 15.60 | 22.30 | 31.20 |
| Meglumin-Starter | 12.00 | 12.00 | 12.00 |
| Gliquidone | 2.57 | 4.38 | 8.26 |
| Povidone K90 | 0.64 | 1.10 | 2.07 |
| Talkum | 1.29 | 2.19 | 4.13 |
| Isopropanol (nur zur Herstellung) | 66.82 | 113.88 | 214.76 |

| Beispiel | c60 | c61 |
|---|---|---|
| Wirkstoffgehalt (Gew.-%): | 17.80 | 27.10 |
| Arginin-Starter | 12.00 | 12.00 |
| Gliquidone | 3.10 | 6.20 |
| Povidone K 90 | 0.78 | 1.55 |
| Talkum | 1.55 | 3.10 |
| Isopropanol (nur zur Herstellung) | 80.60 | 161.20 |

| Beispiel | c62 | c63 | c64 |
|---|---|---|---|
| Wirkstoffgehalt (Gew.-%): | 14.90 | 25.00 | 32.60 |
| Lysin-Starter | 12.00 | 12.00 | 12.00 |
| Gliquidone | 2.41 | 5.32 | 9.12 |
| Povidone K90 | 0.60 | 1.33 | 2.28 |
| Talkum | 1.21 | 2.66 | 4.56 |
| Isopropanol (nur zur Herstellung) | 62.66 | 138.32 | 237.12 |

Repaglinide-Beispiele

| Beispiel | c65 | c66 | c67 |
|---|---|---|---|
| Wirkstoffgehalt (Gew.-%): | 4.30 | 11.00 | 16.50 |
| Neutralpellets | 12.00 | 12.00 | 12.00 |
| Repaglinide | 0.56 | 1,63 | 2.78 |
| Povidone K90 | 0.14 | 0.41 | 0.70 |
| Talkum | 0.28 | 0.82 | 1.39 |
| Isopropanol (nur zur Herstellung) | 14.56 | 42.38 | 72.28 |

| Beispiel | c68 | c69 | c70 |
|---|---|---|---|
| Wirkstoffgehalt (Gew.-%): | 3.40 | 11.40 | 13.50 |
| Meglumin-Starter | 12.00 | 12.00 | 12.00 |
| Repaglinide | 0.44 | 1.70 | 2.12 |
| Povidone K90 | 0.11 | 0.43 | 0.53 |
| Talkum | 0.22 | 0.85 | 1.06 |
| Isopropanol (nur zur Herstellung) | 11.44 | 44.20 | 55.12 |

| Beispiel | c71 | c72 |
|---|---|---|
| Wirkstoffgehalt (Gew.-%): | 5.10 | 8.00 |
| Arginin-Starter | 12.00 | 12.00 |
| Repaglinide | 0.67 | 1.11 |
| Povidone K 90 | 0.17 | 0.28 |
| Talkum | 0.34 | 0.56 |
| Isopropanol (nur zur Herstellung) | 17.42 | 28.86 |

| Beispiel | c73 | c74 | c75 |
|---|---|---|---|
| Wirkstoffgehalt (Gew.-%): | 5.00 | 8.60 | 15.00 |
| Lysin-Starter | 12.00 | 12.00 | 12.00 |
| Repaglinide | 0.66 | 1.21 | 2.44 |
| Povidone K90 | 0.17 | 0.30 | 0.61 |
| Talkum | 0.33 | 0.61 | 1.22 |
| Isopropanol (nur zur Herstellung) | 17.16 | 31.46 | 63.44 |

**Patentansprüche**

1. Pharmazeutische Zusammensetzung mit einer vom Magen-pH weitgehend unabhängigen Bioverfügbarkeit des Wirkstoffs zur oralen Verabreichung von Wirkstoffen mit pH-abhängigen Löslichkeiten und einer Dosenumber größer 1 bei einem pH-Wert von kleiner als 7 umfassend eine Vielzahl von Pellets, die jeweils aus

   a) einem Kernmaterial,
   b) einer optionalen Isolierschicht,
   c) einer Wirkstoffschicht und
   d) einem optionalem Überzug

   aufgebaut sind, **dadurch gekennzeichnet, daß** das Kernmaterial aus einer oder mehreren pharmazeutisch akzeptablen anorganisch oder organischen Base(n) mit einer Wasserlöslichkeit von größer als 1 g / 250 ml bei 20° C, gegebenenfalls unter Zusatz von Bindemitteln oder anderer technologischer Hilfsstoffe, besteht.

2. Pharmazeutische Zusammensetzung mit einer vom Magen-pH weitgehend unabhängigen Bioverfügbarkeit des Wirkstoffs zur oralen Verabreichung von Wirkstoffen mit pH-abhängigen Löslichkeiten und einer Dosenumber größer 1 bei einem pH-Wert von kleiner als 6 umfassend eine Vielzahl von Pellets, die jeweils aus

   a) einem Kernmaterial,
   b) einer optionalen Isolierschicht,
   c) einer Wirkstoffschicht und
   d) einem optionalem Überzug

   aufgebaut sind, **dadurch gekennzeichnet, daß** das Kernmaterial aus einer oder mehreren pharmazeutisch akzeptablen anorganisch oder organischen Base(n) mit einer Wasserlöslichkeit von größer als 1 g / 250 ml bei 20° C, gegebenenfalls unter Zusatz von Bindemitteln oder anderer technologischer Hilfsstoffe, besteht.

3. Pharmazeutische Zusammensetzung nach Anspruch 1 oder 2, bei denen die pharmazeutisch akzeptable Base NaOH, KOH, Ca(OH)$_2$, Na$_2$CO$_3$, Ammoniak, Diethanolamin, Meglumin, Lysin, Arginin, Ethanolamin, Piperazin, Triethanolamin oder Trometamol ist.

4. Pharmazeutische Zusammensetzung nach Anspruch 3, **dadurch gekennzeichnet, dass** die pharmazeutisch akzeptable organische Base Meglumin, Lysin, Arginin, Trometamol ist.

5. Pharmazeutische Zusammensetzung nach Anspruch 4, **dadurch gekennzeichnet, dass** die pharmazeutisch akzeptable organische Base Meglumin ist.

6. Pharmazeutische Zusammensetzung nach einem der vorangehenden Ansprüche, **dadurch gekennzeichnet, dass** der Wirkstoff Telmisartan, Meloxicam, DT-TX 30 SE, BIBV 308 SE (Terbogrel), AGEE 623 (Repaglinide), Gliquidon oder Glibenclamide oder ein physiologisch unbedenkliches Salz davon ist.

7. Pharmazeutische Zusammensetzung nach einem der vorangehenden Ansprüche, bei der das Bindemittel aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat oder aus Kombinationen dieser Polymere besteht.

8. Pharmazeutische Zusammensetzung nach einem der vorangehenden Ansprüche, bei der das Kernmaterial eine mittlere Partikelgröße von 0,4 bis 1,5 mm aufweist.

9. Pharmazeutische Zusammensetzung nach Anspruch 1, bei der die Isolierschicht aus einem wasserlöslichen Polymer, ggf. unter Zusatz geeigneter Weichmacher, Trennmittel und Pigmente, besteht.

10. Pharmazeutische Zusammensetzung nach Anspruch 9, bei der das wasserlösliche Polymer aus Gummi arabicum oder einem partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder aus Kombinationen dieser Polymere besteht.

11. Pharmazeutische Zusammensetzung nach Anspruch 1, bei der der Überzug aus Filmbildnern, Weichmachern und ggf. Pigmenten besteht.

12. Pharmazeutische Zusammensetzung nach einem der vorangehenden Ansprüche, bei der die wirkstoffhaltige Pellets in Hartkapseln abgefüllt werden.

13. Verfahren zur Herstellung einer oral zu applizierenden pharmazeutischen Zusammensetzung enthaltend einen Wirkstoff mit pH-abhängigen Löslichkeitsverhalten und einer Dosenumber von deutlich größer als 1 bei einem pH-Wert von kleiner als 7 oder eines seiner physiologisch verträglichen Salze, umfassend die Schritte:

   a) Aufbau des Kernmaterials aus einer oder mehreren pharmazeutisch akzeptablen anorganischen oder organischen Base(n) mit einer Wasserlöslichkeit von größer als 1 g / 250 ml bei 20° C, gegebenenfalls unter Zusatz von Bindemitteln oder.anderer technologischer Hilfsstoffe, in Kesselverfahren, auf Pelletiertellern oder mittels Extrusion / Sphäronisierung,
   b) Auftragen einer Isolierschicht bestehend aus einem oder mehreren wasserlöslichen, pharmazeutisch akzeptablen Polymeren gegebenenfalls unter Zusatz von Weichmachern, Trennmitteln und/oder Pigmenten auf das Kernmaterial,
   c) Auftragen des Wirkstoffs aus einer bindemittel- und gegebenenfalls trennmittelhaltigen Dispersion und gleichzeitiges und/oder anschließendes Trocknen zur Entfernung des Dispersiönsmittels,
   d) gegebenenfalls Aufbringen eines Überzugs aus Filmbildnern, Weichmachern und gegebenenfalls Pigmenten und
   e) Abfüllung der so erhaltenen wirkstoffhaltigen Pellets in Hartkapseln.

**Claims**

1. Pharmaceutical composition with a bioavailability of the active substance which is substantially independent of the gastric pH, for oral administration of active substances with pH-dependent solubilities and a dose number of more than 1 at a pH of less than 7, comprising a plurality of pellets synthesised in each case from

   a) a core material,
   b) an optional insulating layer,
   c) an active substance layer and
   d) an optional coating ,

   **characterised in that** the core material consists of one or more pharmaceutically acceptable inorganic or organic base(s) with a water solubility of more than 1 g / 250 ml at 20° C, optionally with the addition of binders or other technological adjuvants.

2. Pharmaceutical composition with a bioavailability of the active substance which is substantially independent of the gastric pH, for oral administration of active substances with pH-dependent solubilities and a dose number of more than 1 at a pH of less than 6, comprising a plurality of pellets synthesised in each case from

   a) a core material,
   b) an optional insulating layer,
   c) an active substance layer and
   d) an optional coating ,

   **characterised in that** the core material consists of one or more pharmaceutically acceptable inorganic or organic base(s) with a water solubility of more than 1 g / 250 ml at 20° C, optionally with the addition of binders or other technological adjuvants.

3. Pharmaceutical composition according to claim 1 or 2, wherein the pharmaceutically acceptable base is NaOH, KOH, $Ca(OH)_2$, $Na_2CO_3$, ammonia, diethanolamine, meglumine, lysine, arginine, ethanolamine, piperazine, triethanolamine or trometamol.

4. Pharmaceutical composition according to claim 3, **characterised in that** the pharmaceutically acceptable organic base is meglumine, lysine, arginine, trometamol.

5. Pharmaceutical composition according to claim 4, **characterised in that** the pharmaceutically acceptable organic base is meglumine.

6. Pharmaceutical composition according to one of the preceding claims, **characterised in that** the active substance is telmisartan, meloxicam, DT-TX 30 SE, BIBV 308 SE (terbogrel), AGEE 623 (repaglinide), gliquidone or glibenclamide or a physiologically acceptable salt thereof.

7. Pharmaceutical composition according to one of the preceding claims, wherein the binder is selected from the group comprising the hydroxypropylcelluloses, the hydroxypropylmethylcelluloses, the methylcelluloses, the hydroxyethylcelluloses, the carboxymethylcelluloses, the polyvinylpyrrolidones, the copolymers of N-vinylpyrrolidone and vinyl acetate or combinations of these polymers.

8. Pharmaceutical composition according to one of the preceding claims, wherein the core material has an average particle size of 0.4 to 1.5 mm.

9. Pharmaceutical composition according to claim 1, wherein the insulating layer consists of a water-soluble polymer, optionally with the addition of suitable plasticisers, separating agents and pigments.

10. Pharmaceutical composition according to claim 9, wherein the water-soluble polymer consists of gum arabic or a partially or totally synthetic polymer selected from among the hydroxypropylcelluloses, the hydroxypropylmethylcelluloses, the methylcelluloses, the hydroxyethylcelluloses, the carboxymethylcelluloses, the polyvinylpyrrolidones, the copolymers of N-vinylpyrrolidone and vinyl acetate or combinations of these polymers.

**11.** Pharmaceutical composition according to claim 1, wherein the coating consists of film-forming agents, plasticisers and optionally pigments.

**12.** Pharmaceutical composition according to one of the preceding claims, wherein the pellets containing active substance are packed into hard capsules.

**13.** Process for preparing a pharmaceutical composition for oral administration containing an active substance with pH-dependent solubility characteristics and a dose number of significantly more than 1 at a pH of less than 7 or one of the physiologically acceptable salts thereof, comprising the steps of:

a) synthesising the core material from one or more pharmaceutically acceptable inorganic or organic base(s) with a water solubility of more than 1 g/250 ml at 20° C, optionally with the addition of binders or other technological adjuvants, in by pan methods, on pelleting plates or by extrusion/spheronisation,
b) applying an insulating layer consisting of one or more water-soluble, pharmaceutically acceptable polymers, optionally with the addition of plasticisers, separating agents and/or pigments, to the core material,
c) applying the active substance from a dispersion containing binder and optionally separating agent, and simultaneously or subsequently drying to eliminate the dispersing agent,
d) optionally applying a coating of film-forming agents, plasticisers and optionally pigments and
e) packing the pellets containing active substance thus obtained into hard capsules.

**Revendications**

**1.** Composition pharmaceutique avec une biodisponibilité du principe actif sensiblement indépendante du pH de l'estomac pour l'administration orale de principes actifs ayant des solubilités dépendantes du pH et un nombre de doses supérieur à 1 à un pH inférieur à 7 comprenant une multiplicité de pastilles qui sont formées chacune à partir de

a) un matériau de noyau,
b) une couche isolante éventuelle,
c) une couche de principe actif et
d) un enrobage éventuel

**caractérisée en ce que** le matériau de noyau consiste en une ou plusieurs bases inorganiques ou organiques pharmaceutiquement acceptables ayant une solubilité dans l'eau supérieure à 1 g/250 ml à 20°C, éventuellement avec addition de liants ou d'autres adjuvants technologiques.

**2.** Composition pharmaceutique avec une biodisponibilité du principe actif sensiblement indépendante du pH de l'estomac pour l'administration orale de principes actifs ayant des solubilités dépendantes du pH et un nombre de doses supérieur à 1 à un pH inférieur à 6 comprenant une multiplicité de pastilles qui sont formées chacune à partir de

a) un matériau de noyau,
b) une couche isolante éventuelle,
c) une couche de principe actif et
d) un enrobage éventuel

**caractérisée en ce que** le matériau de noyau consiste en une ou plusieurs bases inorganiques ou organiques pharmaceutiquement acceptables ayant une solubilité dans l'eau supérieure à 1 g/250 ml à 20°C, éventuellement avec addition de liants ou d'autres adjuvants technologiques.

**3.** Composition pharmaceutique selon la revendication 1 ou 2 où la base pharmaceutiquement acceptable est NaOH, KOH, $Ca(OH)_2$, $Na_2CO_3$, l'ammoniac, la diéthanolamine, la méglumine, la lysine, l'arginine, l'éthanolamine, la pipérazine, la triéthanolamine ou le trométamol.

**4.** Composition pharmaceutique selon la revendication 3 **caractérisée en ce que** la base organique pharmaceutiquement acceptable est la méglumine, la lysine, l'arginine, le trométamol.

**5.** Composition pharmaceutique selon la revendication 4 **caractérisée en ce que** la base organique pharmaceutiquement acceptable est la méglumine.

6. Composition pharmaceutique selon l'une des revendications précédentes **caractérisée en ce que** le principe actif est le telmisartan, le meloxicam, DT-TX 30 SE, BIBV 308 SE (terbogrel), AGEE 623 (repaglinide), la gliquidone ou le glibenclamide ou un sel physiologiquement acceptable de ceux-ci.

7. Composition pharmaceutique selon l'une des revendications précédentes où le liant est du groupe des hydroxypropylcelluloses, des hydroxypropylméthylcelluloses, des méthylcelluloses, des hydroxyéthylcelluloses, des carboxyméthylcelluloses, des polyvinylpyrrolidones, des copolymères de N-vinylpyrrolidone et d'acétate de vinyle ou des combinaisons de ces polymères.

8. Composition pharmaceutique selon l'une des revendications précédentes où le matériau de noyau présente une taille de particule moyenne de 0,4 à 1,5 mm.

9. Composition pharmaceutique selon la revendication 1 où la couche isolante consiste en un polymère soluble dans l'eau, éventuellement avec addition de plastifiants, agents de séparation et pigments appropriés.

10. Composition pharmaceutique selon la revendication 9 où le polymère soluble dans l'eau consiste en gomme arabique ou un polymère partiellement ou totalement synthétique du groupe des hydroxypropylcelluloses, des hydroxypropylméthylcelluloses, des méthylcelluloses, des hydroxyéthylcelluloses, des carboxyméthylcelluloses, des polyvinylpyrrolidones, des copolymères de N-vinylpyrrolidone et d'acétate de vinyle ou en combinaisons de ces polymères.

11. Composition pharmaceutique selon la revendication 1 où l'enrobage consiste en filmogènes, plastifiants et éventuellement pigments.

12. Composition pharmaceutique selon l'une des revendications précédentes où les pastilles contenant un principe actif sont introduites dans des capsules dures.

13. Procédé pour produire une composition pharmaceutique destinée à être appliquée par voie orale contenant un principe actif ayant un comportement de solubilité dépendant du pH et un nombre de doses nettement supérieur à 1 à un pH inférieur à 7 ou l'un de ses sels physiologiquement acceptables, comprenant les étapes :

a) formation du matériau de noyau à partir d'une ou plusieurs bases inorganiques ou organiques pharmaceutiquement acceptables ayant une solubilité dans l'eau supérieure à 1 g/250 ml à 20°C, éventuellement avec addition de liants ou d'autres adjuvants technologiques, dans des procédés en pot, sur des plateaux de pastillage ou par extrusion/sphéronisation,
b) application d'une couche isolante consistant en un ou plusieurs polymères pharmaceutiquement acceptables solubles dans l'eau éventuellement avec addition de plastifiants, agents de séparation et/ou pigments sur le matériau de noyau,
c) application du principe actif à partir d'une dispersion contenant un liant et éventuellement un agent de séparation et séchage simultané et/ou subséquent pour retirer le dispersant,
d) éventuellement application d'un enrobage constitué par des filmogènes, des plastifiants et éventuellement des pigments et
e) introduction des pastilles contenant un principe actif ainsi obtenues dans des capsules dures.

**Figur 1:**

Dissolution in 0.005M Citratpuffer

**Figur 2:**

**Schematischer Aufbau der pharmazeutischen Zusammensetzung:**

Kernmaterial enthaltend Base

Isolierschicht (optional)

Wirkstoffschicht.

Überzug (optional)

Figur 3:

**Dissolution in 0.005M Citratpuffer**

Legend: Beispiel c3, Beispiel c4, Beispiel c5, Beispiel c1, Beispiel c2

x-axis: Zeit [Min], y-axis: % Freigabe

Figur 4:

**Dissolution in 0,01M Phosphatpuffer**

Legend: Beispiel c33, Beispiel c35, Beispiel c28, Beispiel c34

x-axis: Zeit [min], y-axis: Freigabe %

Figur 5:

Dissolution in 0,01M Phosphatpuffer

Figur 6:

Dissolution in 0,0025M Phosphatpuffer

Figur 7

**Dissolution in 0,0025M Phosphatpuffer**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *Eur. J. Pharm. Biopharm.,* 2000, vol. 50, 3-12